# EUROPEAN PATENT APPLICATION

(11) **EP 4 454 717 A1**
(43) Date of publication of application: **30.10.2024**
(21) Application number: 22911400.4
(22) Date of filing: 23.12.2022
(51) Int. Cl.: A61Q 19/00, A61K 9/12, A61K 8/89, C09K 23/54

(54) **FROTHING METHOD**

(30) Priority: 24.12.2021 JP 2021211546
(71) Applicant: Kao Corporation, Tokyo 103-8210 (JP)
(72) Inventor: NAGAI, Go, Wakayama-shi, Wakayama 640-8580 (JP); UCHIDA, Yohei, Wakayama-shi, Wakayama 640-8580 (JP); SAKAIHARA, Yuji, Wakayama-shi, Wakayama 640-8580 (JP); HORIE, Yu, Tokyo 131-8501 (JP)
(74) Representative: Hoffmann Eitle
(86) International application number: PCT/JP2022/047604
(87) International publication number: WO 2023/120699

(57) **Abstract**

Provided are: [1] a foaming method including: a step (X) of mixing a foamable composition containing a foaming agent (A) having a time required for a reduction in dynamic surface tension thereof to start of 60,000 ms or less, which is measured by a maximum bubble pressure method, and an organic solvent (B), and a gas, and foaming the composition; and a step (Y) of reducing the size of foam with a porous body; and [2] an article including: a foam discharge container; and a foamable composition in the foam discharge container, wherein the foamable composition contains a foaming agent (A) having a time required for a reduction in dynamic surface tension thereof to start of 60,000 ms or less, which is measured by a maximum bubble pressure method, and an organic solvent (B), and wherein the foam discharge mechanism of the foam discharge container includes a porous body.

## Description

### Technical Field

The present invention relates to a foaming method and an article.

### Background Art

A composition to be used in, for example, perfume, a cosmetic, a skin-care product, or a toiletry product may be blended with a foaming agent from the viewpoint of increasing its volume with foam to improve its properties, such as uniform applicability, an anti-dripping property, a shape-retaining property, and softness of touch.

In addition, the composition may be blended with an organic solvent for improving its applicability.

Technologies each concerning a foamable composition containing a foaming agent and an organic solvent are, for example, technologies described in WO 2009/003946 A1 (Patent Literature 1) and US 2009/0010862 A1 (Patent Literature 2).

In Patent Literature 1, there is a description that a foamable transparent composition that can foam by being mixed with air and is free of a propellant, the composition containing a water-immiscible solvent and a siloxane resin foam stabilizer, may be used in cosmetic applications.

In Patent Literature 2, there is a description that a personal care composition, which contains an oil and a siloxane resin foam stabilizer, and is substantially free of a surfactant and a propellant, can foam, and is clear and non-greasy.

### Summary of Invention

The present invention relates to the following items [1] and [2].
[1] A foaming method, comprising:
   a step (X) of mixing a foamable composition containing a foaming agent (A) having a time required for a reduction in dynamic surface tension thereof to start of 60,000 ms or less, which is measured by the following measurement method 1, and an organic solvent (B), and a gas, and foaming the composition; and
   a step (Y) of reducing a size of foam with a porous body:
      (Measurement Method 1)
   the foaming agent (A) is dissolved in the organic solvent (B) to prepare a solution of the foaming agent (A) having a concentration of 0.5 mass%; then, a dynamic surface tension of the solution of the foaming agent (A) and that of the organic solvent (B) alone at a bubble production time of from 10 ms to 60,000 ms and 25°C are each measured by a maximum bubble pressure method; and then, a first time at which a value obtained by subtracting the dynamic surface tension of the solution of the foaming agent (A) from the dynamic surface tension of the organic solvent (B) alone becomes 0.3 mN/m or more is defined as the time (ms) required for the reduction in dynamic surface tension to start.
[2] An article, comprising:
   a foam discharge container; and
   a foamable composition in the foam discharge container,
   wherein the foamable composition contains a foaming agent (A) having a time required for a reduction in dynamic surface tension thereof to start of 60,000 ms or less, which is measured by the following measurement method 1, and an organic solvent (B), and
   wherein a foam discharge mechanism of the foam discharge container includes a porous body:
      (Measurement Method 1)
   the foaming agent (A) is dissolved in the organic solvent (B) to prepare a solution of the foaming agent (A) having a concentration of 0.5 mass%; then, a dynamic surface tension of the solution of the foaming agent (A) and that of the organic solvent (B) alone at a bubble production time of from 10 ms to 60,000 ms and 25°C are each measured by a maximum bubble pressure method; and then, a first time at which a value obtained by subtracting the dynamic surface tension of the solution of the foaming agent (A) from the dynamic surface tension of the organic solvent (B) alone becomes 0.3 mN/m or more is defined as the time (ms) required for the reduction in dynamic surface tension to start.

### Description of Embodiments

A foamable composition containing a foaming agent and an organic solvent has been susceptible to improvement in terms of foamability, though its applicability is satisfactory because the organic solvent can be turned into foam.

The inventors of the present invention have found that the foamability of a foamable composition containing a foaming agent satisfying a specific dynamic surface tension characteristic and an organic solvent can be improved by: mixing the composition with a gas to foam the composition; and reducing the size of foam with a porous body.

According to the present invention, there can be provided a foaming method and an article each of which is improved in foamability.

### [Foaming Method]

A foaming method of the present invention includes: a step (X) of mixing a foamable composition containing a foaming agent (A) having a time required for a reduction in dynamic surface tension thereof to start of 60,000 ms or less, which is measured by the following measurement method 1, and an organic solvent (B), and a gas, and foaming the composition; and a step (Y) of reducing the size of foam with a porous body.

### (Measurement Method 1)

The foaming agent (A) is dissolved in the organic solvent (B) to prepare a solution of the foaming agent (A) having a concentration of 0.5 mass%, then, the dynamic surface tension of the solution of the foaming agent (A) and that of the organic solvent (B) alone at a bubble production time of from 10 ms to 60,000 ms and 25°C are each measured by a maximum bubble pressure method, and then, the first time at which a value obtained by subtracting the dynamic surface tension of the solution of the foaming agent (A) from the dynamic surface tension of the organic solvent (B) alone becomes 0.3 mN/m or more is defined as the time (ms) required for the reduction in dynamic surface tension to start.

The term "maximum bubble pressure method" as used herein refers to a method including: supplying a gas to a capillary (probe) inserted into a liquid; measuring the maximum pressure (maximum bubble pressure) when a bubble is produced from the tip of the probe; and calculating a surface tension. The basic principle of the method is based on the Young-Laplace equation.

A solvent identical in kind and composition to the organic solvent (B) for forming the foamable composition according to the present invention serving as an object is used as the organic solvent (B) to be used in the measurement method 1.

Details about a method of measuring a dynamic surface tension are as described in Examples to be described later.

According to the foaming method of the present invention, the foamability of the foamable composition can be improved.

In this description, as foam to be produced becomes more fine-textured and bulkier, the foamability can be judged to be more satisfactory, and as a time period from the production of the foam to its disappearance becomes longer, foam stability can be judged to be more satisfactory.

According to the foaming method of the present invention, the foamability of the foamable composition containing the foaming agent (A) satisfying a specific dynamic surface tension characteristic and the organic solvent (B) can be improved by: mixing the composition with the gas to foam the composition; and reducing the size of the foam with the porous body. Although the reason therefor is uncertain, the reason is conceived as described below.

First, it is conceivable that the time required for the reduction in dynamic surface tension of the foaming agent (A) to start represents the rate at which the dynamic surface tension reduces, and hence represents the rate at which the foaming agent (A) adsorbs to a gas-liquid interface, that is, the formation rate of a gas-liquid interfacial film. In addition, when the composition contains the foaming agent (A) having a time required for the reduction in dynamic surface tension to start of 60,000 ms or less and the organic solvent (B), the formation rate of the gas-liquid interfacial film increases, and hence the rate at which, and the amount in which, the foam is formed increase. In addition, the produced foam can be reduced in size with the porous body. It is conceivable from the foregoing that the foamability can be improved.

### (Foamable Composition)

### <Foaming Agent (A)>

The foamable composition according to the present invention contains the foaming agent (A).

The term "foaming agent" as used herein means a compound having the following action: when the compound is dissolved in a solvent, the compound produces foam in a solution to be obtained, and stabilizes the produced foam to prevent its disappearance.

The foaming agents (A) may be used alone or in combination thereof.

The foaming agent (A) preferably contains a silicone (A1) from the viewpoint of further improving the foamability and foam stability of the foamable composition according to the present invention, and the viewpoint of further improving the durability and water resistance of a coating film obtained from the foamable composition according to the present invention.

From the viewpoint of further improving the foamability and foam stability of the foamable composition according to the present invention, and the viewpoint of further improving the durability and water resistance of the coating film obtained from the foamable composition according to the present invention, the silicone (A1) preferably contains at least one kind selected from the group consisting of: trimethylsiloxysilicate; phenylpropyldimethylsiloxysilicate; fluorine-modified alkyldimethylsiloxysilicate; cross polymers obtained by cross-linking those siloxysilicates with dimethiconol or the like; polydimethylsiloxane; polyhydromethylsiloxane; hydrosilyl-modified silicone; polyether-modified silicone; carboxy-modified silicone; amino-modified silicone; fluoroalkyl-modified silicone; carbinol-modified silicone; phenyl-modified silicone; polyglycerin-modified silicone; higher fatty acid ester-modified silicone; polysiloxane betaine; and polysilicone-9, more preferably contains at least one kind selected from the group consisting of: trimethylsiloxysilicate; polydimethylsiloxane; polyether-modified silicone; carboxy-modified silicone; amino-modified silicone; fluoroalkyl-modified silicone; carbinol-modified silicone; and phenyl-modified silicone, still more preferably contains at least one kind selected from the group consisting of: trimethylsiloxysilicate; polydimethylsiloxane; carboxy-modified silicone; amino-modified silicone; fluoroalkyl-modified silicone; and carbinol-modified silicone, and still more preferably contains at least one kind selected from the group consisting of: trimethylsiloxysilicate; polydimethylsiloxane; polyether-modified silicone; carboxy-modified silicone; and amino-modified silicone.

The polyether-modified silicone has a structure in which an alkyl group in the side chain and/or terminal of a silicone is substituted with a polyether group. The polyether group is suitably at least one kind selected from the group consisting of: a polyethyleneoxy group; a polypropyleneoxy group; and a polyalkyleneoxy group in which an ethyleneoxy (EO) group and a propyleneoxy (PO) group are added blockwise or randomly, and is more preferably a polyethyleneoxy group.

The foaming agent (A) may contain a foaming agent except the silicone (A1) to the extent that the effect of the invention is not impaired. An example of the foaming agent except the silicone (A1) is a polymer surfactant such as a (meth)acrylic polymer.

From the viewpoint of further improving the foamability and foam stability of the foamable composition according to the present invention, the content of the silicone (A1) in the foaming agent (A) is preferably 50 mass% or more, more preferably 60 mass% or more, still more preferably 70 mass% or more, still more preferably 80 mass% or more, still more preferably 90 mass% or more, still more preferably 95 mass% or more, still more preferably 98 mass% or more, and is preferably 100 mass% or less.

The time required for the reduction in dynamic surface tension of the foaming agent (A) to start, which is measured by the measurement method 1, is 60,000 ms or less. However, from the viewpoint of further improving the foamability and foam stability of the foamable composition according to the present invention, the time is preferably 10,000 ms or less, more preferably 6,000 ms or less, still more preferably 3,000 ms or less, still more preferably less than 1,000 ms, still more preferably 800 ms or less, still more preferably 600 ms or less, still more preferably 500 ms or less, still more preferably 250 ms or less, still more preferably 200 ms or less, still more preferably 150 ms or less, still more preferably 100 ms or less, still more preferably 70 ms or less, still more preferably 60 ms or less. Although the lower limit value of the time required for the reduction in dynamic surface tension to start, which is measured by the measurement method 1, is not particularly limited, the lower limit value is, for example, 1 ms or more, and may be 5 ms or more.

From the viewpoint of further improving the foamability and foam stability of the foamable composition according to the present invention, the amount of a reduction in static surface tension of the foaming agent (A), which is measured by the following measurement method 2, is preferably 0.5 mN/m or more, more preferably 0.7 mN/m or more, still more preferably 1.0 mN/m or more, still more preferably 1.5 mN/m or more, still more preferably 1.7 mN/m or more, still more preferably 1.8 mN/m or more, still more preferably 2.0 mN/m or more. Although the upper limit value of the amount of the reduction in static surface tension, which is measured by the following measurement method 2, is not particularly limited, the upper limit value is, for example, 10.0 mN/m or less, and may be 7.0 mN/m or less, may be 5.0 mN/m or less, may be 3.5 mN/m or less, or may be 3.0 mN/m or less.

### (Measurement Method 2)

The foaming agent (A) is dissolved in the organic solvent (B) to prepare a solution of the foaming agent (A) having a concentration of 0.5 mass%, then, the static surface tension of the solution of the foaming agent (A) and that of the organic solvent (B) alone at 25°C are each measured by a Du Nouy method through the lifting of a ring at a speed of 1 mm/min, and then, a value obtained by subtracting the static surface tension of the solution of the foaming agent (A) from the static surface tension of the organic solvent (B) alone is defined as the amount (mN/m) of the reduction in static surface tension.

A solvent identical in kind and composition to the organic solvent (B) for forming the foamable composition according to the present invention serving as an object is used as the organic solvent (B) to be used in the measurement method 2.

Details about a method of measuring a static surface tension are as described in Examples to be described later.

### <Organic Solvent (B)>

The foamable composition according to the present invention contains the organic solvent (B).

The organic solvents (B) may be used alone or in combination thereof.

The organic solvent (B) is preferably in a liquid state at 25°C from the viewpoint of further improving the foamability and foam stability of the foamable composition according to the present invention, and the viewpoint of further improving the applicability thereof to hair, skin, or the like.

The phrase "in a liquid state at 25°C" as used herein refers to a state in which the composition has fluidity in a bulk state under an environment under 1 atm and at 25°C.

From the viewpoint of further improving the foamability of the foamable composition according to the present invention, the surface tension of the organic solvent (B) is preferably 18 mN/m or more, more preferably 22 mN/m or more, still more preferably 24 mN/m or more, and is preferably 35 mN/m or less, more preferably 30 mN/m or less, still more preferably 28 mN/m or less.

The surface tension of the organic solvent (B) is a static surface tension at 25°C measured by the Du Nouy method, and details about the measurement method are as described in Examples to be described later.

From the viewpoint of further improving the foamability of the foamable composition according to the present invention, the shear viscosity of the organic solvent (B) is preferably 40 mPa·s or less, more preferably 32 mPa·s or less, still more preferably 13 mPa·s or less, still more preferably 11 mPa·s or less, and is preferably 0.5 mPa·s or more, more preferably 1.5 mPa·s or more, still more preferably 2.5 mPa·s or more, still more preferably 3.0 mPa·s or more.

Details about a method of measuring the shear viscosity of the organic solvent (B) at 25°C and a shear rate of 500 s⁻¹ are as described in Examples to be described later.

From the viewpoint of further improving the foamability and foam stability of the foamable composition according to the present invention, and the viewpoint of further improving the applicability thereof to hair, skin, or the like, the number of the carbon atoms of the organic solvent (B) is preferably 1 or more, more preferably 2 or more, still more preferably 3 or more, still more preferably 4 or more, still more preferably 5 or more, still more preferably 6 or more, still more preferably 8 or more, and is preferably 30 or less, more preferably 25 or less, still more preferably 22 or less, still more preferably 20 or less, still more preferably 19 or less, still more preferably 18 or less.

From the viewpoint of further improving the foamability and foam stability of the foamable composition according to the present invention, and the viewpoint of further improving the applicability thereof to hair, skin, or the like, the organic solvent (B) preferably contains at least one kind of water-immiscible solvent (B 1) selected from the group consisting of: a hydrocarbon oil; an ester oil; a fatty acid; a vegetable oil; and a mineral oil, and more preferably contains at least one kind of water-immiscible solvent (B1) selected from the group consisting of: a hydrocarbon oil; an ester oil; and a fatty acid.

From the viewpoint of further improving the foamability and foam stability of the foamable composition according to the present invention, and the viewpoint of further improving the applicability to hair, skin, or the like, the hydrocarbon oil preferably contains at least one kind selected from the group consisting of: an α-olefin oligomer; a liquid paraffin; a liquid isoparaffin (e.g., a light liquid isoparaffin or a heavy liquid isoparaffin), such as isodecane, isododecane, isohexadecane, or hydrogenated polyisobutene; liquid ozokerite; squalane; pristane; squalene; and the like. Among them, a liquid isoparaffin is preferred as the hydrocarbon oil, a light liquid isoparaffin is more preferred, at least one kind selected from the group consisting of: isododecane; isohexadecane; and hydrogenated polyisobutene is still more preferred, hydrogenated polyisobutene is still more preferred, hydrogenated polyisobutene having 8 to 22 carbon atoms is still more preferred, hydrogenated polyisobutene having 8 to 18 carbon atoms is still more preferred, and hydrogenated polyisobutene having 14 to 18 carbon atoms is still more preferred from the viewpoint of further improving the foamability and foam stability of the foamable composition according to the present invention, and the viewpoint of further improving the applicability thereof to hair, skin, or the like.

From the viewpoint of further improving the foamability and foam stability of the foamable composition according to the present invention, and the viewpoint of further improving the applicability thereof to hair, skin, or the like, the ester oil preferably contains at least one kind selected from the group consisting of: cetyl 2-ethylhexanoate; stearyl 2-ethylhexanoate; isononyl isononanoate; 2-ethylhexyl isononanoate; isodecyl isononanoate; isotridecyl isononanoate; hexyl laurate; isostearyl laurate; butyl myristate; isopropyl myristate; decyl myristate; isotridecyl myristate; isocetyl myristate; isopropyl palmitate; 2-ethylhexyl palmitate; 2-ethylhexyl stearate; isopropyl isostearate; isodecyl neopentanoate; ethyl oleate; isodecyl oleate; ethyl linoleate; isopropyl linoleate; lanolin acetate; castor oil fatty acid methyl ester (methyl ricinoleate); glyceryl tri(2-ethylhexanoate); glyceryl caprylate; glyceryl tri(caprylate/caprate); neopentyl glycol di(2-ethylhexanoate); propanediol di(caprylate/caprate); and an alkyl benzoate (whose alkyl has 12 to 15 carbon atoms).

The ester oil preferably contains at least one kind selected from the group consisting of: cetyl 2-ethylhexanoate; stearyl 2-ethylhexanoate; isononyl isononanoate; 2-ethylhexyl isononanoate; isodecyl isononanoate; isotridecyl isononanoate; hexyl laurate; isostearyl laurate; butyl myristate; isopropyl myristate; decyl myristate; isotridecyl myristate; isocetyl myristate; isopropyl palmitate; 2-ethylhexyl palmitate; 2-ethylhexyl stearate; isopropyl isostearate; isodecyl neopentanoate; glyceryl caprylate; glyceryl tri(caprylate/caprate); neopentyl glycol di(2-ethylhexanoate); propanediol di(caprylate/caprate); and an alkyl benzoate (whose alkyl has 12 to 15 carbon atoms) among the above-mentioned compounds, more preferably contains at least one kind selected from the group consisting of: isononyl isononanoate; isopropyl myristate; isopropyl palmitate; and 2-ethylhexyl palmitate, still more preferably contains at least one kind selected from the group consisting of: isononyl isononanoate; and 2-ethylhexyl palmitate, and still more preferably contains isononyl isononanoate, from the viewpoint of further improving the foamability and foam stability of the foamable composition according to the present invention, and the viewpoint of further improving the applicability thereof to hair, skin, or the like.

From the viewpoint of further improving the foamability and foam stability of the foamable composition according to the present invention, and the viewpoint of further improving the applicability thereof to hair, skin, or the like, the fatty acid preferably contains at least one kind selected from the group consisting of: 2-ethylhexanoic acid; lauric acid; myristic acid; pentadecanoic acid; palmitic acid; palmitoleic acid; margaric acid; stearic acid; oleic acid; and linoleic acid. Among them, 2-ethylhexanoic acid is preferred as the fatty acid from the viewpoint of further improving the foamability and foam stability of the foamable composition according to the present invention, and the viewpoint of further improving the applicability thereof to hair, skin, or the like.

From the viewpoint of further improving the foamability and foam stability of the foamable composition according to the present invention, and the viewpoint of further improving the applicability thereof to hair, skin, or the like, examples of the vegetable oil include: soybean oil; rice germ oil; rice bran oil; jojoba oil; avocado oil; almond oil; olive oil; cacao butter; sesame oil; persic oil; castor oil; coconut oil; safflower oil; eucalyptus oil; rapeseed oil; and an oil that is obtained by hydrogenating each of those oils and is in a liquid state at 25°C. However, in this description, oils corresponding to the ester oil and the fatty acid are excluded from the vegetable oil.

From the viewpoint of further improving the foamability and foam stability of the foamable composition according to the present invention, and the viewpoint of further improving the applicability thereof to hair, skin, or the like, the mineral oil is, for example, a distillate oil obtained by distilling a paraffin base crude oil, an intermediate base crude oil, or a naphthene base crude oil at normal pressure or by distilling the residual oil of normal-pressure distillation under reduced pressure, or a refined oil (e.g., a solvent-refined oil, a hydrogenated refined oil, a dewaxed oil, or a clay-treated oil) obtained by refining such crude oil in accordance with an ordinary method. However, in this description, an oil corresponding to the hydrocarbon oil is excluded from the mineral oil.

The water-immiscible solvent (B1) is preferably immiscible with water at 25°C from the viewpoint of further improving the foamability and foam stability of the foamable composition according to the present invention.

The phrase "immiscible with water" as used herein means that there is a ratio at which the solvent does not form any solution with water.

From the viewpoint of further improving the foamability and foam stability of the foamable composition according to the present invention, the content of the water-immiscible solvent (B1) in the organic solvent (B) is preferably 50 mass% or more, more preferably 60 mass% or more, still more preferably 70 mass% or more, still more preferably 80 mass% or more, still more preferably 90 mass% or more, still more preferably 95 mass% or more, still more preferably 98 mass% or more, and is preferably 100 mass% or less.

From the viewpoint of further improving the foamability and foam stability of the foamable composition according to the present invention, and the viewpoint of further improving the applicability thereof to hair, skin, or the like, the shear viscosity of the foamable composition according to the present invention at 25°C and a shear rate of 500 s⁻¹ is preferably 22.0 mPa·s or less, more preferably 20.0 mPa·s or less, still more preferably 18.0 mPa·s or less, still more preferably 16.0 mPa·s or less, still more preferably 15.0 mPa·s or less, still more preferably 14.0 mPa·s or less. Although the lower limit value of the shear viscosity is not particularly limited, the lower limit value is, for example, 0.1 mPa·s or more, and may be 0.5 mPa·s or more, may be 1.0 mPa·s or more, may be 2.0 mPa·s or more, or may be 2.5 mPa·s or more.

Details about a method of measuring the shear viscosity at 25°C and a shear rate of 500 s⁻¹ are as described in Examples to be described later.

From the viewpoint of further improving the foamability and foam stability of the foamable composition according to the present invention, and the viewpoint of further improving the durability and water resistance of a coating film obtained from the foamable composition according to the present invention, the content of the foaming agent (A) in the foamable composition according to the present invention is preferably 0.01 mass% or more, more preferably 0.05 mass% or more, still more preferably 0.1 mass% or more, still more preferably 0.3 mass% or more, still more preferably 0.5 mass% or more, still more preferably 1.0 mass% or more, still more preferably 2.0 mass% or more, still more preferably 3.0 mass% or more, still more preferably 4.0 mass% or more, still more preferably 6.0 mass% or more, still more preferably 8.0 mass% or more when the total amount of the foamable composition is defined as 100 mass%. From the viewpoint of further improving the applicability of the composition to hair, skin, or the like, the content is preferably 50 mass% or less, more preferably 40 mass% or less, still more preferably 30 mass% or less, still more preferably 20 mass% or less, still more preferably 18 mass% or less, still more preferably 15 mass% or less, still more preferably 12 mass% or less.

From the viewpoint of further improving the applicability to hair, skin, or the like, the content of the organic solvent (B) in the foamable composition according to the present invention is preferably 50 mass% or more, more preferably 60 mass% or more, still more preferably 70 mass% or more, still more preferably 80 mass% or more, still more preferably 82 mass% or more, still more preferably 85 mass% or more, still more preferably 88 mass% or more when the total amount of the foamable composition is defined as 100 mass%. From the viewpoint of further improving the foamability and foam stability of the foamable composition according to the present invention, and the viewpoint of further improving the durability and water resistance of the coating film obtained from the foamable composition according to the present invention, the content is preferably 99.99 mass% or less, more preferably 99.95 mass% or less, still more preferably 99.9 mass% or less, still more preferably 99.7 mass% or less, still more preferably 99.5 mass% or less, still more preferably 99.0 mass% or less, still more preferably 98.0 mass% or less, still more preferably 97.0 mass% or less, still more preferably 96.0 mass% or less, still more preferably 94.0 mass% or less, still more preferably 92.0 mass% or less.

From the viewpoint of further improving the foamability and foam stability of the foamable composition according to the present invention, and the viewpoint of further improving the durability and water resistance of the coating film obtained from the foamable composition according to the present invention, the mass ratio ((A)/(B)) of the content of the foaming agent (A) in the foamable composition according to the present invention to the content of the organic solvent (B) therein is preferably 0.001 or more, more preferably 0.01 or more, still more preferably 0.03 or more, still more preferably 0.05 or more, still more preferably 0.08 or more. From the viewpoint of further improving the applicability of the composition to hair, skin, or the like, the mass ratio is preferably 1 or less, more preferably 0.5 or less, still more preferably 0.3 or less, still more preferably 0.2 or less, still more preferably 0.15 or less.

From the viewpoint of further improving the foamability and foam stability of the foamable composition according to the present invention, the viewpoint of further improving the durability and water resistance of the coating film obtained from the foamable composition according to the present invention, and the viewpoint of further improving the applicability to hair, skin, or the like, the content of water in the foamable composition according to the present invention is preferably less than 50 mass%, more preferably less than 25 mass%, still more preferably less than 10 mass%, still more preferably less than 5 mass%, still more preferably less than 1 mass%, still more preferably less than 0.1 mass%, still more preferably less than 0.01 mass% when the total amount of the foamable composition is defined as 100 mass%, and it is preferred that the foamable composition according to the present invention be substantially free of water. The phrase "substantially free of water" as used herein means that no water is intentionally added, and the phrase does not exclude the presence of a small amount of water serving as an impurity.

The foamable composition according to the present invention may be used in, for example, all kinds of perfume and cosmetics each utilizing the formation of foam.

The term "perfume and cosmetics each utilizing the formation of foam" as used herein refers to a product to be used by being foamed among, for example, the following products: products to be brought into direct contact with a human body, such as perfume, a cosmetic, a skin-care product, and a toiletry product; and daily commodities, such as a laundry detergent, a household detergent, a fabric softener, an insecticide, an office glue, a paint, an aromatic, and a pet shampoo.

The foamable composition according to the present invention may appropriately contain, as a component except the foaming agent (A) and the organic solvent (B), a component to be used in accordance with the kinds of perfume and cosmetics to the extent that the effect of the present invention is not impaired. Examples of such component include sugars, an alcohol, a vitamin, an amino acid, a UV absorber, an oil, a water-soluble polymer, a thickener, a neutralizer, a pH adjuster, a microbicide, an anti-inflammatory agent, a preservative, a coloring agent, a chelating agent, a whitening agent, a blood circulation promoter, a cooling agent, an antiperspirant, an insect repellent, a physiologically active ingredient, salts, a moisturizer, an antioxidant, a fragrance, a plant extract, and a drug (excluding those corresponding to the foaming agent (A) and the organic solvent (B)).

Examples of the form of the foamable composition according to the present invention include a liquid, an emulsion, a cream, a paste, and a gel. The foamable composition according to the present invention may also be applied as a stock solution of a spray, a mousse, or the like.

From the viewpoint of further improving the foamability and foam stability of the foamable composition according to the present invention, and the viewpoint of further improving the durability and water resistance of the coating film obtained from the foamable composition according to the present invention, the total content of the foaming agent (A) and the organic solvent (B) in the foamable composition according to the present invention is preferably 30 mass% or more, more preferably 40 mass% or more, still more preferably 50 mass% or more, still more preferably 60 mass% or more, still more preferably 70 mass% or more, still more preferably 80 mass% or more, still more preferably 90 mass% or more, still more preferably 95 mass% or more, and is preferably 100 mass% or less, when the total amount of the foamable composition is defined as 100 mass%.

The content of the component except the foaming agent (A) and the organic solvent (B) in the foamable composition according to the present invention is not particularly limited because the content is set in accordance with the kinds of perfume and cosmetics. The content may be appropriately set on the basis of various kinds of known information.

A method of producing the foamable composition according to the present invention is not particularly limited, and the composition may be produced by, for example, mixing the respective components with a known apparatus.

### (Step (X))

The foaming method of the present invention includes the step (X) of mixing the foamable composition according to the present invention and the gas, and foaming the composition.

A method of mixing the foamable composition and the gas to foam the composition is, for example, a method including using a foam discharge container. In the method including using the foam discharge container, the foamable composition is loaded into the foam discharge container, and the loaded foamable composition is mixed with the gas such as air in the discharge container to produce foam. The foam thus produced passes through the internal flow path of the foam discharge container, and is discharged from a discharge port formed in the discharge head thereof.

The internal flow path (foam discharge mechanism) of the foam discharge container preferably has a porous body through which the foam passes. Thus, the step (Y) may be performed simultaneously or continuously with the step (X) by using the foam discharge container.

Any container may be used as the foam discharge container as long as the loaded foamable composition can be discharged as foam by being mixed with the gas such as air in the discharge container. Examples thereof include: such a pump foamer container that the pressing of its pump head pressurizes the cylinder of an air chamber and a cylinder storing the foamable composition to mix the air and the foamable composition, to thereby discharge the composition in a foam state; such a squeeze foamer container that the pressing of the body portion of the container that can be deformed by pressing deforms the container to discharge the composition; and a trigger spray container that is used by pressing the head of a cap including a pump mechanism with a finger.

A non-gas type foam discharge container is preferred as the foam discharge container from the viewpoint of the ease with which the container is recycled and in terms of environmental friendliness because no propellant is used.

The term "non-gas type foam discharge container" as used herein means a non-aerosol type foam discharge container except an aerosol type container using a propellant, and refers to a discharge container that can mix the composition loaded thereinto and air to discharge the composition in a foam state. Specific examples thereof include a pump foamer container, a squeeze foamer container, and a trigger spray container.

### (Step (Y))

The foaming method of the present invention includes the step (Y) of reducing the size of the foam with the porous body.

Herein, in the foaming method according to the present invention, the step (X) and the step (Y) may be simultaneously performed, or the step (Y) may be performed after the performance of the step (X).

From the viewpoint of further improving the foamability of the foamable composition, a porous membrane body, such as a mesh-shaped, a sponge-like, or a sintered porous membrane body, is preferred as the porous body, and a mesh-shaped porous membrane body is more preferred.

In addition, although a material for the porous body is not particularly limited, the porous body is preferably made of a thermoplastic resin from the viewpoint of easily forming a porous shape. The thermoplastic resin is preferably a resin that is excellent in strength, heat resistance, and chemical resistance, and examples thereof include polyamide, polyester, polyolefin, a fluororesin, and a silicone resin.

From the viewpoint of further improving the foamability, the opening ratio of the porous body is preferably 30% or more, more preferably 35% or more, still more preferably 38% or more, still more preferably 40% or more, still more preferably 42% or more. From the same viewpoint, the opening ratio is preferably 80% or less, more preferably 70% or less, still more preferably 60% or less, still more preferably 55% or less.

The opening ratio of the porous body may be calculated from the ratio of the total area of the opening portions thereof to the total surface area of the porous body. Opening ratio (%) = (total area of opening portions)/(total surface area of porous body) × 100

From the viewpoint of further improving the foamability, the aperture diameter of the porous body is preferably 70 µm or more, more preferably 100 µm or more, still more preferably 120 µm or more, still more preferably 140 µm or more, still more preferably 150 µm or more, still more preferably 168 µm or more. From the same viewpoint, the aperture diameter is preferably 1,000 µm or less, more preferably 500 µm or less, still more preferably 400 µm or less, still more preferably 350 µm or less.

For example, when the porous body has a lattice-like mesh shape, the aperture diameter of the porous body is a distance between wires adjacent to each other among wires for forming the mesh, that is, the length of one side of an opening. In addition, when an opening portion of the porous body has a circular shape, the aperture diameter of the porous body is the diameter of the opening portion.

The composition after its foaming obtained through the step (X) and the step (Y) may be preferably used by being applied to an object, such as hair or skin.

### [Article]

An article of the present invention is an article including: a foam discharge container; and a foamable composition in the foam discharge container, wherein the foamable composition contains a foaming agent (A) having a time required for a reduction in dynamic surface tension thereof to start of 60,000 ms or less, which is measured by the following measurement method 1, and an organic solvent (B), and wherein a foam discharge mechanism of the foam discharge container includes a porous body.

### (Measurement Method 1)

The foaming agent (A) is dissolved in the organic solvent (B) to prepare a solution of the foaming agent (A) having a concentration of 0.5 mass%, then, the dynamic surface tension of the solution of the foaming agent (A) and that of the organic solvent (B) alone at a bubble production time of from 10 ms to 60,000 ms and 25°C are each measured by a maximum bubble pressure method, and then, the first time at which a value obtained by subtracting the dynamic surface tension of the solution of the foaming agent (A) from the dynamic surface tension of the organic solvent (B) alone becomes 0.3 mN/m or more is defined as the time (ms) required for the reduction in dynamic surface tension to start.

According to the article of the present invention, while the foamable composition is foamed by mixing the foamable composition and a gas such as air with the foam discharge container, foam is discharged via the foam discharge mechanism including the porous body. Thus, the foam can be reduced in size.

The article of the present invention can obtain the same effect as that of the foaming method according to the present invention because of the following reasons: the article includes the foamable composition according to the present invention in the foam discharge container; and while the foamable composition is foamed by mixing the composition and the gas with the foam discharge container, the foam can be reduced in size via the foam discharge mechanism including the porous body.

A preferred aspect of the foamable composition is the same as a preferred aspect of the foamable composition in the foaming method according to the present invention described above, and a preferred aspect of the foam discharge container is the same as a preferred aspect of the foam discharge container in the foaming method according to the present invention described above.

Specific examples of the article of the present invention include a pump foamer formulation, a squeeze foamer formulation, and a trigger spray formulation. In addition, examples of the applications of those formulations include perfume, a cosmetic, a skin-care product, a toiletry product, a laundry detergent, a household detergent, a fabric softener, an insecticide, an office glue, a paint, an aromatic, and a pet shampoo.

Regarding the above-mentioned embodiments, the present invention further discloses the following embodiment modes.
<1> A foaming method, including:
   a step (X) of mixing a foamable composition containing a foaming agent (A) having a time required for a reduction in dynamic surface tension thereof to start of 60,000 ms or less, which is measured by the following measurement method 1, and an organic solvent (B), and a gas, and foaming the composition; and
   a step (Y) of reducing a size of foam with a porous body:
      (Measurement Method 1)
   the foaming agent (A) is dissolved in the organic solvent (B) to prepare a solution of the foaming agent (A) having a concentration of 0.5 mass%; then, a dynamic surface tension of the solution of the foaming agent (A) and that of the organic solvent (B) alone at a bubble production time of from 10 ms to 60,000 ms and 25°C are each measured by a maximum bubble pressure method; and then, a first time at which a value obtained by subtracting the dynamic surface tension of the solution of the foaming agent (A) from the dynamic surface tension of the organic solvent (B) alone becomes 0.3 mN/m or more is defined as the time (ms) required for the reduction in dynamic surface tension to start.
<2> The foaming method according to Item <1>, wherein the time required for the reduction in dynamic surface tension of the foaming agent (A) to start, which is measured by the measurement method 1, is preferably 10,000 ms or less, more preferably 6,000 ms or less, still more preferably 3,000 ms or less, still more preferably less than 1,000 ms, still more preferably 800 ms or less, still more preferably 600 ms or less, still more preferably 500 ms or less, still more preferably 250 ms or less, still more preferably 200 ms or less, still more preferably 150 ms or less, still more preferably 100 ms or less, still more preferably 70 ms or less, still more preferably 60 ms or less, and may be 1 ms or more, or may be 5 ms or more.
<3> The foaming method according to Item <1> or <2>, wherein the amount of a reduction in static surface tension of the foaming agent (A), which is measured by the following measurement method 2, is preferably 0.5 mN/m or more, more preferably 0.7 mN/m or more, still more preferably 1.0 mN/m or more, still more preferably 1.5 mN/m or more, still more preferably 1.7 mN/m or more, still more preferably 1.8 mN/m or more, still more preferably 2.0 mN/m or more, and may be 10.0 mN/m or less, may be 7.0 mN/m or less, may be 5.0 mN/m or less, may be 3.5 mN/m or less, or may be 3.0 mN/m or less:
   (Measurement Method 2)
   the foaming agent (A) is dissolved in the organic solvent (B) to prepare a solution of the foaming agent (A) having a concentration of 0.5 mass%; then, a static surface tension of the solution of the foaming agent (A) and that of the organic solvent (B) alone at 25°C are each measured by a Du Nouy method through lifting of a ring at a speed of 1 mm/min; and then, a value obtained by subtracting the static surface tension of the solution of the foaming agent (A) from the static surface tension of the organic solvent (B) alone is defined as the amount (mN/m) of the reduction in static surface tension.
<4> The foaming method according to any one of Items <1> to <3>, wherein the foaming agent (A) contains a silicone (A1).
<5> The foaming method according to any one of Items <1> to <4>, wherein the silicone (A1) preferably contains at least one kind selected from the group consisting of: trimethylsiloxysilicate; phenylpropyldimethylsiloxysilicate; fluorine-modified alkyldimethylsiloxysilicate; cross polymers obtained by cross-linking those siloxysilicates with dimethiconol or the like; polydimethylsiloxane; polyhydromethylsiloxane; hydrosilyl-modified silicone; polyether-modified silicone; carboxy-modified silicone; amino-modified silicone; fluoroalkyl-modified silicone; carbinol-modified silicone; phenyl-modified silicone; polyglycerin-modified silicone; higher fatty acid ester-modified silicone; polysiloxane betaine; and polysilicone-9, and more preferably contains at least one kind selected from the group consisting of: trimethylsiloxysilicate; polydimethylsiloxane; polyether-modified silicone; carboxy-modified silicone; amino-modified silicone; fluoroalkyl-modified silicone; carbinol-modified silicone; and phenyl-modified silicone, still more preferably contains at least one kind selected from the group consisting of: trimethylsiloxysilicate; polydimethylsiloxane; carboxy-modified silicone; amino-modified silicone; fluoroalkyl-modified silicone; and carbinol-modified silicone, and still more preferably contains at least one kind selected from the group consisting of: trimethylsiloxysilicate; polydimethylsiloxane; polyether-modified silicone; carboxy-modified silicone; and amino-modified silicone.
<6> The foaming method according to any one of Items <1> to <5>, wherein the content of the silicone (A1) in the foaming agent (A) is preferably 50 mass% or more, more preferably 60 mass% or more, still more preferably 70 mass% or more, still more preferably 80 mass% or more, still more preferably 90 mass% or more, still more preferably 95 mass% or more, still more preferably 98 mass% or more, and is preferably 100 mass% or less.
<7> The foaming method according to any one of Items <1> to <6>, wherein the static surface tension of the organic solvent (B) at 25°C, which is measured by a Du Nouy method, is preferably 18 mN/m or more, more preferably 22 mN/m or more, still more preferably 24 mN/m or more, and is preferably 35 mN/m or less, more preferably 30 mN/m or less, still more preferably 28 mN/m or less.
<8> The foaming method according to any one of Items <1> to <7>, wherein the shear viscosity of the organic solvent (B) at 25°C and a shear rate of 500 s⁻¹ is preferably 40 mPa·s or less, more preferably 32 mPa·s or less, still more preferably 13 mPa·s or less, still more preferably 11 mPa·s or less, and is preferably 0.5 mPa·s or more, more preferably 1.5 mPa·s or more, still more preferably 2.5 mPa·s or more, still more preferably 3.0 mPa·s or more.
<9> The foaming method according to any one of Items <1> to <8>, wherein the number of the carbon atoms of the organic solvent (B) is preferably 1 or more, more preferably 2 or more, still more preferably 3 or more, still more preferably 4 or more, still more preferably 5 or more, still more preferably 6 or more, still more preferably 8 or more, and is preferably 30 or less, more preferably 25 or less, still more preferably 22 or less, still more preferably 20 or less, still more preferably 19 or less, still more preferably 18 or less.
<10> The foaming method according to any one of Items <1> to <9>, wherein the organic solvent (B) contains at least one kind of water-immiscible solvent (B 1) selected from the group consisting of: a hydrocarbon oil; an ester oil; and a fatty acid.
<11> The foaming method according to Item <10>, wherein the hydrocarbon oil preferably contains at least one kind selected from the group consisting of: an α-olefin oligomer; a liquid paraffin; a liquid isoparaffin; liquid ozokerite; squalane; pristane; and squalene, and is more preferably a liquid isoparaffin, still more preferably a light liquid isoparaffin, still more preferably at least one kind selected from the group consisting of: isododecane; isohexadecane; and hydrogenated polyisobutene, still more preferably hydrogenated polyisobutene, still more preferably hydrogenated polyisobutene having 8 to 22 carbon atoms, still more preferably hydrogenated polyisobutene having 8 to 18 carbon atoms, still more preferably hydrogenated polyisobutene having 14 to 18 carbon atoms.
<12> The foaming method according to Item <10> or <11>, wherein the ester oil preferably contains at least one kind selected from the group consisting of: cetyl 2-ethylhexanoate; stearyl 2-ethylhexanoate; isononyl isononanoate; 2-ethylhexyl isononanoate; isodecyl isononanoate; isotridecyl isononanoate; hexyl laurate; isostearyl laurate; butyl myristate; isopropyl myristate; decyl myristate; isotridecyl myristate; isocetyl myristate; isopropyl palmitate; 2-ethylhexyl palmitate; 2-ethylhexyl stearate; isopropyl isostearate; isodecyl neopentanoate; ethyl oleate; isodecyl oleate; ethyl linoleate; isopropyl linoleate; lanolin acetate; castor oil fatty acid methyl ester (methyl ricinoleate); glyceryl tri(2-ethylhexanoate); glyceryl caprylate; glyceryl tri(caprylate/caprate); neopentyl glycol di(2-ethylhexanoate); propanediol di(caprylate/caprate); and an alkyl benzoate (whose alkyl has 12 to 15 carbon atoms), more preferably contains at least one kind selected from the group consisting of: cetyl 2-ethylhexanoate; stearyl 2-ethylhexanoate; isononyl isononanoate; 2-ethylhexyl isononanoate; isodecyl isononanoate; isotridecyl isononanoate; hexyl laurate; isostearyl laurate; butyl myristate; isopropyl myristate; decyl myristate; isotridecyl myristate; isocetyl myristate; isopropyl palmitate; 2-ethylhexyl palmitate; 2-ethylhexyl stearate; isopropyl isostearate; isodecyl neopentanoate; glyceryl caprylate; glyceryl tri(caprylate/caprate); neopentyl glycol di(2-ethylhexanoate); propanediol di(caprylate/caprate); and an alkyl benzoate (whose alkyl has 12 to 15 carbon atoms), still more preferably contains at least one kind selected from the group consisting of: isononyl isononanoate; isopropyl myristate; isopropyl palmitate; and 2-ethylhexyl palmitate, and still more preferably contains at least one kind selected from the group consisting of: isononyl isononanoate; and 2-ethylhexyl palmitate.
<13> The foaming method according to any one of Items <10> to <12>, wherein the fatty acid preferably contains at least one kind selected from the group consisting of: 2-ethylhexanoic acid; lauric acid; myristic acid; pentadecanoic acid; palmitic acid; palmitoleic acid; margaric acid; stearic acid; oleic acid; and linoleic acid, and is more preferably 2-ethylhexanoic acid.
<14> The foaming method according to any one of Items <10> to <13>, wherein the content of the water-immiscible solvent (B1) in the organic solvent (B) is preferably 50 mass% or more, more preferably 60 mass% or more, still more preferably 70 mass% or more, still more preferably 80 mass% or more, still more preferably 90 mass% or more, still more preferably 95 mass% or more, still more preferably 98 mass% or more, and is preferably 100 mass% or less.
<15> The foaming method according to any one of Items <1> to <14>, wherein the shear viscosity of the foamable composition at 25°C and a shear rate of 500 s⁻¹ is preferably 22.0 mPa·s or less, more preferably 20.0 mPa·s or less, still more preferably 18.0 mPa·s or less, still more preferably 16.0 mPa·s or less, still more preferably 15.0 mPa·s or less, still more preferably 14.0 mPa·s or less, and may be 0.1 mPa·s or more, may be 0.5 mPa·s or more, may be 1.0 mPa·s or more, may be 2.0 mPa·s or more, or may be 2.5 mPa·s or more.
<16> The foaming method according to any one of Items <1> to <15>, wherein the content of the foaming agent (A) in the foamable composition is preferably 0.01 mass% or more, more preferably 0.05 mass% or more, still more preferably 0.1 mass% or more, still more preferably 0.3 mass% or more, still more preferably 0.5 mass% or more, still more preferably 1.0 mass% or more, still more preferably 2.0 mass% or more, still more preferably 3.0 mass% or more, still more preferably 4.0 mass% or more, still more preferably 6.0 mass% or more, still more preferably 8.0 mass% or more, and is preferably 50 mass% or less, more preferably 40 mass% or less, still more preferably 30 mass% or less, still more preferably 20 mass% or less, still more preferably 18 mass% or less, still more preferably 15 mass% or less, still more preferably 12 mass% or less, when the total amount of the foamable composition is defined as 100 mass%.
<17> The foaming method according to any one of Items <1> to <16>, wherein the content of the organic solvent (B) in the foamable composition is preferably 50 mass% or more, more preferably 60 mass% or more, still more preferably 70 mass% or more, still more preferably 80 mass% or more, still more preferably 82 mass% or more, still more preferably 85 mass% or more, still more preferably 88 mass% or more, and is preferably 99.99 mass% or less, more preferably 99.95 mass% or less, still more preferably 99.9 mass% or less, still more preferably 99.7 mass% or less, still more preferably 99.5 mass% or less, still more preferably 99.0 mass% or less, still more preferably 98.0 mass% or less, still more preferably 97.0 mass% or less, still more preferably 96.0 mass% or less, still more preferably 94.0 mass% or less, still more preferably 92.0 mass% or less, when the total amount of the foamable composition is defined as 100 mass%.
<18> The foaming method according to any one of Items <1> to <17>, wherein the mass ratio ((A)/(B)) of the content of the foaming agent (A) in the foamable composition to the content of the organic solvent (B) therein is preferably 0.001 or more, more preferably 0.01 or more, still more preferably 0.03 or more, still more preferably 0.05 or more, still more preferably 0.08 or more, and is preferably 1 or less, more preferably 0.5 or less, still more preferably 0.3 or less, still more preferably 0.2 or less, still more preferably 0.15 or less.
<19> The foaming method according to any one of Items <1> to <18>, wherein the opening ratio of the porous body is preferably 30% or more, more preferably 35% or more, still more preferably 38% or more, still more preferably 40% or more, still more preferably 42% or more, and is preferably 80% or less, more preferably 70% or less, still more preferably 60% or less, still more preferably 55% or less.
<20> The foaming method according to any one of Items <1> to <19>, wherein the aperture diameter of the porous body is preferably 70 µm or more, more preferably 100 µm or more, still more preferably 120 µm or more, still more preferably 140 µm or more, still more preferably 150 µm or more, still more preferably 168 µm or more, and is preferably 1,000 µm or less, more preferably 500 µm or less, still more preferably 400 µm or less, still more preferably 350 µm or less.
<21> The foaming method according to any one of Items <1> to <20>, wherein the content of water in the foamable composition is preferably less than 50 mass%, more preferably less than 25 mass%, still more preferably less than 10 mass%, still more preferably less than 5 mass%, still more preferably less than 1 mass%, still more preferably less than 0.1 mass%, still more preferably less than 0.01 mass% when the total amount of the foamable composition is defined as 100 mass%, and it is still more preferred that the foamable composition be substantially free of water.
<22> The foaming method according to any one of Items <1> to <21>, wherein the total content of the foaming agent (A) and the organic solvent (B) in the foamable composition is preferably 30 mass% or more, more preferably 40 mass% or more, still more preferably 50 mass% or more, still more preferably 60 mass% or more, still more preferably 70 mass% or more, still more preferably 80 mass% or more, still more preferably 90 mass% or more, still more preferably 95 mass% or more, and is preferably 100 mass% or less, when the total amount of the foamable composition is defined as 100 mass%.
<23> The foaming method according to any one of Items <1> to <22>, wherein the step (X) includes mixing the foamable composition and the gas with a foam discharge container to foam the composition.
<24> The foaming method according to Item <23>, wherein the foam discharge container is a pump foamer container, a squeeze foamer container, or a trigger spray container.
<25> The foaming method according to any one of Items <1> to <24>, wherein the porous body is preferably a mesh-shaped, sponge-like, or sintered porous membrane body, more preferably a mesh-shaped porous membrane body.
<26> The foaming method according to any one of Items <1> to <25>, wherein a material for the porous body is preferably polyamide, polyester, polyolefin, a fluororesin, or a silicone resin.
<27> An article, including:
   a foam discharge container; and
   a foamable composition in the foam discharge container,
   wherein the foamable composition contains a foaming agent (A) having a time required for a reduction in dynamic surface tension thereof to start of 60,000 ms or less, which is measured by the following measurement method 1, and an organic solvent (B), and
   wherein a foam discharge mechanism of the foam discharge container includes a porous body:
      (Measurement Method 1)
   the foaming agent (A) is dissolved in the organic solvent (B) to prepare a solution of the foaming agent (A) having a concentration of 0.5 mass%; then, a dynamic surface tension of the solution of the foaming agent (A) and that of the organic solvent (B) alone at a bubble production time of from 10 ms to 60,000 ms and 25°C are each measured by a maximum bubble pressure method; and then, a first time at which a value obtained by subtracting the dynamic surface tension of the solution of the foaming agent (A) from the dynamic surface tension of the organic solvent (B) alone becomes 0.3 mN/m or more is defined as the time (ms) required for the reduction in dynamic surface tension to start.
<28> The article according to Item <27>, wherein the time required for the reduction in dynamic surface tension of the foaming agent (A) to start, which is measured by the measurement method 1, is preferably 10,000 ms or less, more preferably 6,000 ms or less, still more preferably 3,000 ms or less, still more preferably less than 1,000 ms, still more preferably 800 ms or less, still more preferably 600 ms or less, still more preferably 500 ms or less, still more preferably 250 ms or less, still more preferably 200 ms or less, still more preferably 150 ms or less, still more preferably 100 ms or less, still more preferably 70 ms or less, still more preferably 60 ms or less, and may be 1 ms or more, or may be 5 ms or more.
<29> The article according to Item <27> or <28>, wherein the amount of a reduction in static surface tension of the foaming agent (A), which is measured by the following measurement method 2, is preferably 0.5 mN/m or more, more preferably 0.7 mN/m or more, still more preferably 1.0 mN/m or more, still more preferably 1.5 mN/m or more, still more preferably 1.7 mN/m or more, still more preferably 1.8 mN/m or more, still more preferably 2.0 mN/m or more, and may be 10.0 mN/m or less, may be 7.0 mN/m or less, may be 5.0 mN/m or less, may be 3.5 mN/m or less, or may be 3.0 mN/m or less:
   (Measurement Method 2)
   the foaming agent (A) is dissolved in the organic solvent (B) to prepare a solution of the foaming agent (A) having a concentration of 0.5 mass%; then, a static surface tension of the solution of the foaming agent (A) and that of the organic solvent (B) alone at 25°C are each measured by a Du Nouy method through lifting of a ring at a speed of 1 mm/min; and then, a value obtained by subtracting the static surface tension of the solution of the foaming agent (A) from the static surface tension of the organic solvent (B) alone is defined as the amount (mN/m) of the reduction in static surface tension.
<30> The article according to any one of Items <27> to <29>, wherein the foaming agent (A) contains a silicone (A1).
<31> The article according to any one of Items <27> to <30>, wherein the silicone (A1) preferably contains at least one kind selected from the group consisting of: trimethylsiloxysilicate; phenylpropyldimethylsiloxysilicate; fluorine-modified alkyldimethylsiloxysilicate; cross polymers obtained by cross-linking those siloxysilicates with dimethiconol or the like; polydimethylsiloxane; polyhydromethylsiloxane; hydrosilyl-modified silicone; polyether-modified silicone; carboxy-modified silicone; amino-modified silicone; fluoroalkyl-modified silicone; carbinol-modified silicone; phenyl-modified silicone; polyglycerin-modified silicone; higher fatty acid ester-modified silicone; polysiloxane betaine; and polysilicone-9, more preferably contains at least one kind selected from the group consisting of: trimethylsiloxysilicate; polydimethylsiloxane; polyether-modified silicone; carboxy-modified silicone; amino-modified silicone; fluoroalkyl-modified silicone; carbinol-modified silicone; and phenyl-modified silicone, still more preferably contains at least one kind selected from the group consisting of: trimethylsiloxysilicate; polydimethylsiloxane; carboxy-modified silicone; amino-modified silicone; fluoroalkyl-modified silicone; and carbinol-modified silicone, and still more preferably contains at least one kind selected from the group consisting of: trimethylsiloxysilicate; polydimethylsiloxane; polyether-modified silicone; carboxy-modified silicone; and amino-modified silicone.
<32> The article according to any one of Items <27> to <31>, wherein the content of the silicone (A1) in the foaming agent (A) is preferably 50 mass% or more, more preferably 60 mass% or more, still more preferably 70 mass% or more, still more preferably 80 mass% or more, still more preferably 90 mass% or more, still more preferably 95 mass% or more, still more preferably 98 mass% or more, and is preferably 100 mass% or less.
<33> The article according to any one of Items <27> to <32>, wherein the static surface tension of the organic solvent (B) at 25°C, which is measured by a Du Nouy method, is preferably 18 mN/m or more, more preferably 22 mN/m or more, still more preferably 24 mN/m or more, and is preferably 35 mN/m or less, more preferably 30 mN/m or less, still more preferably 28 mN/m or less.
<34> The article according to any one of Items <27> to <33>, wherein the shear viscosity of the organic solvent (B) at 25°C and a shear rate of 500 s⁻¹ is preferably 40 mPa·s or less, more preferably 32 mPa·s or less, still more preferably 13 mPa·s or less, still more preferably 11 mPa·s or less, and is preferably 0.5 mPa·s or more, more preferably 1.5 mPa·s or more, still more preferably 2.5 mPa·s or more, still more preferably 3.0 mPa·s or more.
<35> The article according to any one of Items <27> to <34>, wherein the number of the carbon atoms of the organic solvent (B) is preferably 1 or more, more preferably 2 or more, still more preferably 3 or more, still more preferably 4 or more, still more preferably 5 or more, still more preferably 6 or more, still more preferably 8 or more, and is preferably 30 or less, more preferably 25 or less, still more preferably 22 or less, still more preferably 20 or less, still more preferably 19 or less, still more preferably 18 or less.
<36> The article according to any one of Items <27> to <35>, wherein the organic solvent (B) contains at least one kind of water-immiscible solvent (B 1) selected from the group consisting of: a hydrocarbon oil; an ester oil; and a fatty acid.
<37> The article according to Item <36>, wherein the hydrocarbon oil preferably contains at least one kind selected from the group consisting of: an α-olefin oligomer; a liquid paraffin; a liquid isoparaffin; liquid ozokerite; squalane; pristane; and squalene, and is more preferably a liquid isoparaffin, still more preferably a light liquid isoparaffin, still more preferably at least one kind selected from the group consisting of: isododecane; isohexadecane; and hydrogenated polyisobutene, still more preferably hydrogenated polyisobutene, still more preferably hydrogenated polyisobutene having 8 to 22 carbon atoms, still more preferably hydrogenated polyisobutene having 8 to 18 carbon atoms, still more preferably hydrogenated polyisobutene having 14 to 18 carbon atoms.
<38> The article according to Item <36> or <37>, wherein the ester oil preferably contains at least one kind selected from the group consisting of: cetyl 2-ethylhexanoate; stearyl 2-ethylhexanoate; isononyl isononanoate; 2-ethylhexyl isononanoate; isodecyl isononanoate; isotridecyl isononanoate; hexyl laurate; isostearyl laurate; butyl myristate; isopropyl myristate; decyl myristate; isotridecyl myristate; isocetyl myristate; isopropyl palmitate; 2-ethylhexyl palmitate; 2-ethylhexyl stearate; isopropyl isostearate; isodecyl neopentanoate; ethyl oleate; isodecyl oleate; ethyl linoleate; isopropyl linoleate; lanolin acetate; castor oil fatty acid methyl ester (methyl ricinoleate); glyceryl tri(2-ethylhexanoate); glyceryl caprylate; glyceryl tri(caprylate/caprate); neopentyl glycol di(2-ethylhexanoate); propanediol di(caprylate/caprate); and an alkyl benzoate (whose alkyl has 12 to 15 carbon atoms), more preferably contains at least one kind selected from the group consisting of: cetyl 2-ethylhexanoate; stearyl 2-ethylhexanoate; isononyl isononanoate; 2-ethylhexyl isononanoate; isodecyl isononanoate; isotridecyl isononanoate; hexyl laurate; isostearyl laurate; butyl myristate; isopropyl myristate; decyl myristate; isotridecyl myristate; isocetyl myristate; isopropyl palmitate; 2-ethylhexyl palmitate; 2-ethylhexyl stearate; isopropyl isostearate; isodecyl neopentanoate; glyceryl caprylate; glyceryl tri(caprylate/caprate); neopentyl glycol di(2-ethylhexanoate); propanediol di(caprylate/caprate); and an alkyl benzoate (whose alkyl has 12 to 15 carbon atoms), still more preferably contains at least one kind selected from the group consisting of: isononyl isononanoate; isopropyl myristate; isopropyl palmitate; and 2-ethylhexyl palmitate, and still more preferably contains at least one kind selected from the group consisting of: isononyl isononanoate; and 2-ethylhexyl palmitate.
<39> The article according to any one of Items <36> to <38>, wherein the fatty acid preferably contains at least one kind selected from the group consisting of: 2-ethylhexanoic acid; lauric acid; myristic acid; pentadecanoic acid; palmitic acid; palmitoleic acid; margaric acid; stearic acid; oleic acid; and linoleic acid, and is more preferably 2-ethylhexanoic acid.
<40> The article according to any one of Items <36> to <39>, wherein the content of the water-immiscible solvent (B1) in the organic solvent (B) is preferably 50 mass% or more, more preferably 60 mass% or more, still more preferably 70 mass% or more, still more preferably 80 mass% or more, still more preferably 90 mass% or more, still more preferably 95 mass% or more, still more preferably 98 mass% or more, and is preferably 100 mass% or less.
<41> The article according to any one of Items <36> to <40>, wherein the shear viscosity of the foamable composition at 25°C and a shear rate of 500 s⁻¹ is preferably 22.0 mPa·s or less, more preferably 20.0 mPa·s or less, still more preferably 18.0 mPa·s or less, still more preferably 16.0 mPa·s or less, still more preferably 15.0 mPa·s or less, still more preferably 14.0 mPa·s or less, and may be 0.1 mPa·s or more, may be 0.5 mPa·s or more, may be 1.0 mPa·s or more, may be 2.0 mPa·s or more, or may be 2.5 mPa·s or more.
<42> The article according to any one of Items <27> to <41>, wherein the content of the foaming agent (A) in the foamable composition is preferably 0.01 mass% or more, more preferably 0.05 mass% or more, still more preferably 0.1 mass% or more, still more preferably 0.3 mass% or more, still more preferably 0.5 mass% or more, still more preferably 1.0 mass% or more, still more preferably 2.0 mass% or more, still more preferably 3.0 mass% or more, still more preferably 4.0 mass% or more, still more preferably 6.0 mass% or more, still more preferably 8.0 mass% or more, and is preferably 50 mass% or less, more preferably 40 mass% or less, still more preferably 30 mass% or less, still more preferably 20 mass% or less, still more preferably 18 mass% or less, still more preferably 15 mass% or less, still more preferably 12 mass% or less, when the total amount of the foamable composition is defined as 100 mass%.
<43> The article according to any one of Items <27> to <42>, wherein the content of the organic solvent (B) in the foamable composition is preferably 50 mass% or more, more preferably 60 mass% or more, still more preferably 70 mass% or more, still more preferably 80 mass% or more, still more preferably 82 mass% or more, still more preferably 85 mass% or more, still more preferably 88 mass% or more, and is preferably 99.99 mass% or less, more preferably 99.95 mass% or less, still more preferably 99.9 mass% or less, still more preferably 99.7 mass% or less, still more preferably 99.5 mass% or less, still more preferably 99.0 mass% or less, still more preferably 98.0 mass% or less, still more preferably 97.0 mass% or less, still more preferably 96.0 mass% or less, still more preferably 94.0 mass% or less, still more preferably 92.0 mass% or less, when the total amount of the foamable composition is defined as 100 mass%.
<44> The article according to any one of Items <27> to <43>, wherein the mass ratio ((A)/(B)) of the content of the foaming agent (A) in the foamable composition to the content of the organic solvent (B) therein is preferably 0.001 or more, more preferably 0.01 or more, still more preferably 0.03 or more, still more preferably 0.05 or more, still more preferably 0.08 or more, and is preferably 1 or less, more preferably 0.5 or less, still more preferably 0.3 or less, still more preferably 0.2 or less, still more preferably 0.15 or less.
<45> The article according to any one of Items <27> to <44>, wherein the opening ratio of the porous body is preferably 30% or more, more preferably 35% or more, still more preferably 38% or more, still more preferably 40% or more, still more preferably 42% or more, and is preferably 80% or less, more preferably 70% or less, still more preferably 60% or less, still more preferably 55% or less.
<46> The article according to any one of Items <27> to <45>, wherein the aperture diameter of the porous body is preferably 70 µm or more, more preferably 100 µm or more, still more preferably 120 µm or more, still more preferably 140 µm or more, still more preferably 150 µm or more, still more preferably 168 µm or more, and is preferably 1,000 µm or less, more preferably 500 µm or less, still more preferably 400 µm or less, still more preferably 350 µm or less.
<47> The article according to any one of Items <27> to <46>, wherein the content of water in the foamable composition is preferably less than 50 mass%, more preferably less than 25 mass%, still more preferably less than 10 mass%, still more preferably less than 5 mass%, still more preferably less than 1 mass%, still more preferably less than 0.1 mass%, still more preferably less than 0.01 mass% when the total amount of the foamable composition is defined as 100 mass%, and it is still more preferred that the foamable composition be substantially free of water.
<48> The article according to any one of Items <27> to <47>, wherein the total content of the foaming agent (A) and the organic solvent (B) in the foamable composition is preferably 30 mass% or more, more preferably 40 mass% or more, still more preferably 50 mass% or more, still more preferably 60 mass% or more, still more preferably 70 mass% or more, still more preferably 80 mass% or more, still more preferably 90 mass% or more, still more preferably 95 mass% or more, and is preferably 100 mass% or less, when the total amount of the foamable composition is defined as 100 mass%.
<49> The article according to any one of Items <27> to <48>, wherein the foam discharge container is a non-gas type foam discharge container.
<50> The article according to Item <49>, wherein the foam discharge container is a pump foamer container, a squeeze foamer container, or a trigger spray container.
<51> The article according to any one of Items <27> to <50>, wherein the porous body is preferably a mesh-shaped, sponge-like, or sintered porous membrane body, more preferably a mesh-shaped porous membrane body.
<52> The article according to any one of Items <27> to <51>, wherein a material for the porous body is preferably polyamide, polyester, polyolefin, a fluororesin, or a silicone resin.

### Examples

The present invention is specifically described below by way of Examples. However, the present invention is by no means limited to Examples. In the following Examples and Comparative Examples, the terms "part(s)" and "%" mean "part(s) by mass" and "mass%", respectively, unless otherwise specified.

In Examples and Comparative Examples, the following foaming agents and organic solvents were used.

### ·Foaming agent

### (Silicone)

Unmodified silicone 1 (trimethylsiloxysilicate, manufactured by Wacker Asahikasei Silicone Co., Ltd., BELSII, (trademark) TMS 803)
Unmodified silicone 2 (polydimethylsiloxane, manufactured by Shin-Etsu Chemical Co., Ltd., product name: KF-96-20cs)
Polyether-modified silicone 1 (functional group: polyoxyalkylene group, PEG-9 polydimethylsiloxyethyl dimethicone, manufactured by Shin-Etsu Chemical Co., Ltd., product name: KF-6028)
Polyether-modified silicone 2 (functional group: polyoxyalkylene group, PEG-3 dimethicone, manufactured by Shin-Etsu Chemical Co., Ltd., product name: KF-6015)
Carboxy-modified silicone 1 (functional group: carboxy group, biscarboxydecyl dimethicone, manufactured by Momentive, product name: Silsoft^{™} INX Fluid)
Amino-modified silicone 1 (functional group: amino group, amino-modified silicone, manufactured by Shin-Etsu Chemical Co., Ltd., product name: KF-864)
Long-chain alkyl-modified silicone 1 (functional group: alkyl group, long-chain alkyl-modified silicone, manufactured by Shin-Etsu Chemical Co., Ltd., product name: KF-412)

### ·Organic solvent

### (Water-immiscible Solvent that is in Liquid State at 25°C)

Hydrocarbon oil 1 (hydrogenated polyisobutene, manufactured by NOF Corporation, product name: PARLEAM (trademark) 3, carbon number: 12)
Hydrocarbon oil 2 (hydrogenated polyisobutene, manufactured by NOF Corporation, product name: PARLEAM (trademark) 4, carbon number: 16)
Hydrocarbon oil 3 (hydrogenated polyisobutene, manufactured by NOF Corporation, product name: PARLEAM (trademark) EX, carbon number: 20)
Hydrocarbon oil 4 (hydrogenated polyisobutene, manufactured by NOF Corporation, product name: PARLEAM (trademark) 6, carbon number: 24)
Ester oil 1 (isononyl isononanoate, The Nisshin OilliO Group, Ltd., product name: SALACOS 99, carbon number: 18)
Ester oil 2 (2-ethylhexyl palmitate, The Nisshin OilliO Group, Ltd., product name: SALACOS P-8, carbon number: 24)
Fatty acid 1 (2-ethylhexanoic acid, manufactured by FUJIFILM Wako Pure Chemical Corporation, carbon number: 8)

The various physical properties of the foaming agents were measured by the following methods.

### (1) Dynamic Surface Tension of Foaming Agent

A foaming agent was dissolved in an organic solvent to prepare a foaming agent solution having a concentration of 0.5 mass%. Then, the dynamic surface tension of the resultant foaming agent solution and that of the organic solvent alone at a bubble production time of from 10 ms to 60,000 ms and 25°C were each measured with a bubble pressure type dynamic surface tensiometer "BP100 (product name)" manufactured by KRUSS GmbH by a maximum bubble pressure method. Then, the first time at which a value obtained by subtracting the dynamic surface tension of the foaming agent solution from the dynamic surface tension of the organic solvent alone became 0.3 mN/m or more was calculated as a time (ms) required for a reduction in dynamic surface tension of the foaming agent to start.

Herein, solvents identical in kind and composition to the organic solvents used in Examples and Comparative Example were each used as the organic solvent.

A glass capillary was used after the following: the capillary was treated by being immersed in Silanization solution I (manufactured by Sigma-Aldrich Co. LLC) for 12 hours or more, and its tip portion was lightly shaved with a file.

### (2) Static Surface Tension of Foaming Agent

A foaming agent was dissolved in an organic solvent to prepare a foaming agent solution having a concentration of 0.5 mass%. Then, the static surface tension of the foaming agent solution and that of the organic solvent alone at 25°C were each measured with a surface tensiometer "K100 (product name)" manufactured by KRUSS GmbH by a Du Nouy method through the lifting of a ring at a speed of 1 mm/min. Then, a value obtained by subtracting the static surface tension of the foaming agent solution from the static surface tension of the organic solvent alone was calculated as the amount (mN/m) of a reduction in static surface tension of the foaming agent.

Herein, solvents identical in kind and composition to the organic solvents used in Examples and Comparative Example were each used as the organic solvent.

The various physical properties of the organic solvent (B) were measured by the following methods.

### (1) Surface Tension of Organic Solvent (B)

The static surface tension of the solvent at 25°C was measured with a surface tensiometer "K100 (product name)" manufactured by KRUSS GmbH by the Du Nouy method through the lifting of a ring at a speed of 1 mm/min.

### (2) Shear Viscosity of Organic Solvent (B)

The shear viscosity of the solvent at 25°C and a shear rate of 500 s⁻¹ was measured with a rheometer under the following conditions.

| | |
|---|---|
| Equipment: | MCR-302 (manufactured by Anton Paar GmbH) |
| Jig: | CP50-1 (diameter: 50 mm) |
| Temperature: | 25°C |
| Shear rate: | 500 s⁻¹ |

### Example 1

The unmodified silicone 1 (1 g) serving as a foaming agent and the hydrocarbon oil 1 (19 g) serving as an organic solvent were loaded into a 30-milliliter container, and were shaken and mixed for 10 minutes to provide a foamable composition.

### Examples 2 to 19 and Comparative Example 1

Foamable compositions were each prepared in the same manner as in Example 1 except that: the kinds of the foaming agent and the organic solvent were changed to those shown in Table 1; and the concentration of the foaming agent was changed to a value shown in Table 1.

### [Physical Property Measurement and Evaluation]

The foamable compositions obtained in Examples and Comparative Example were each subjected to the following physical property measurement and evaluations. The evaluation results are shown in Table 1.

### <Shear Viscosity of Foamable Composition>

The shear viscosity of the foamable composition at 25°C and a shear rate of 500 s⁻¹ was measured with a rheometer under the following conditions.

| | |
|---|---|
| Equipment: | MCR-302 (manufactured by Anton Paar GmbH) |
| Jig: | CP50-1 (diameter: 50 mm) |
| Temperature: | 25°C |
| Shear rate: | 500 s⁻¹ |

### <Evaluation of Foamability>

The foamable compositions were each foamed with a pump foamer container manufactured by Yoshino Kogyosho Co., Ltd. Herein, a mechanism obtained by mounting two porous membrane bodies each made of nylon having a mesh size of 90/inch (aperture diameter: 177 µm, opening ratio: 39%) was used as a foam discharge mechanism. Next, the appearance of the produced foam was observed, and the foamable composition was evaluated for its foamability on the basis of the following evaluation criteria.

### (Evaluation Criteria)

1: Liquid-like foam
2: Foam containing many fine bubbles
3: Foam that is fine and slightly bulky
4: Foam that is fine-textured and bulky
5: Foam that is extremely fine-textured and extremely bulky

### <Evaluation of Foam Stability>

The foamable compositions were each foamed with a pump foamer container manufactured by Yoshino Kogyosho Co., Ltd. Herein, a mechanism obtained by mounting two porous membrane bodies each made of nylon having a mesh size of 90/inch (aperture diameter: 177 µm, opening ratio: 39%) was used as a foam discharge mechanism. Next, the foamable composition was evaluated for its foam stability by measuring a time period from the production of the foam to its disappearance.

**Table 1**

| | | (A) Foaming agent | | | (B) Organic solvent | | | Foamable composition | | | Evaluation | |
|---|---|---|---|---|---|---|---|---|---|---|---|---|
| | | Kind | Amount of reduction in static surface tension | Time required for reduction in dynamic surface tension to start | Kind | Surface tension | Shear viscosity | Foaming agent concentration | (A)/(B) | Shear viscosity | Foamability | Stability |
| | | | [mN/m] | [ms] | | [mN/m] | [mPa·s] | | | [mPa·s] | | [min] |
| Example | 1 | Unmodified silicone 1 | 2.2 | 10 | Hydrocarbon oil 1 | 23.5 | 1.21 | 5% | 0.05 | 1.13 | 4 | - |
| | 2 | Unmodified silicone 1 | 2.3 | 50 | Hydrocarbon oil 2 | 24.9 | 3.26 | 5% | 0.05 | 3.77 | 5 | - |
| | 3 | Unmodified silicone 1 | 2.3 | 50 | Hydrocarbon | 24.9 | 3.26 | 10% | 0.11 | 4.18 | 5 | 50 |
| | 4 | Polyether-modified silicone 1 | 2.1 | 10 | Hydrocarbon oil 2 | 24.9 | 3.26 | 10% | 0.11 | 8.67 | 5 | 15 |
| | 5 | Carboxy-modified silicone 1 | 2.2 | 10 | Hydrocarbon oil 2 | 24.9 | 3.26 | 10% | 0.11 | 6.37 | 5 | 10 |
| | 6 | Amino-modified silicone 1 | 1.8 | 10 | Hydrocarbon oil 2 | 24.9 | 3.26 | 10% | 0.11 | 8.30 | 5 | 4 |
| | 7 | Unmodified silicone 2 | 0.9 | 10 | Hydrocarbon oil 2 | 24.9 | 3.26 | 10% | 0.11 | 2.88 | 5 | 2.5 |
| | 8 | Unmodified silicone 1 | 4.4 | 80 | Hydrocarbon oil 3 | 26.3 | 14.0 | 5% | 0.05 | 14.7 | 3 | - |
| | 9 | Unmodified silicone 1 | 4.4 | 80 | Hydrocarbon oil 3 | 26.3 | 14.0 | 15% | 0.18 | 21.2 | 3 | - |
| | 10 | Unmodified silicone 1 | 5.3 | 50 | Hydrocarbon oil 4 | 25.9 | 32.2 | 5% | 0.05 | 35.8 | 2 | - |
| | 11 | Unmodified silicone 1 | 2.0 | 40 | Ester oil 1 | 24.8 | 5.31 | 5% | 0.05 | 5.37 | 5 | - |
| | 12 | Unmodified silicone 1 | 2.0 | 40 | Ester oil 1 | 24.8 | 5.31 | 10% | 0.11 | 7.15 | 5 | 60 |
| | 13 | Carboxy-modified silicone 1 | 2.9 | 10 | Ester oil 1 | 24.8 | 5.31 | 10% | 0.11 | 8.38 | 5 | 40 |
| | 14 | Amino-modified silicone 1 | 2.3 | 10 | Ester oil 1 | 24.8 | 5.31 | 10% | 0.11 | 13.0 | 5 | 15 |
| | 15 | Unmodified silicone 2 | 1.4 | 10 | Ester oil 1 | 24.8 | 5.31 | 10% | 0.11 | 6.14 | 5 | - |
| | 16 | Polyether-modified silicone 1 | 1.5 | 50 | Ester oil 1 | 24.8 | 5.31 | 10% | 0.11 | 8.70 | 4 | - |
| | 17 | Polyether-modified silicone 2 | 1.0 | 400 | Ester oil 1 | 24.8 | 5.31 | 10% | 0.11 | 7.34 | 3 | - |
| | 18 | Unmodified silicone 1 | 5.2 | 10 | Ester oil 2 | 28.8 | 11.3 | 5% | 0.05 | 12.5 | 4 | - |
| | 19 | Unmodified silicone 1 | 3.2 | 60 | Fatty acid 1 | 27.0 | 5.87 | 5% | 0.05 | 6.88 | 5 | - |
| Comparative Example | 1 | Long-chain alkyl-modified silicone 1 | 0.1 | >60,000 | Hydrocarbon oil 2 | 24.9 | 3.26 | 10% | 0.11 | 5.76 | 1 | - |

### Examples 20 to 27

The respective evaluations were performed in the same manner as in Example 2 except that the aperture diameter of each of the porous membrane bodies arranged in the internal flow path of the foam discharge container was changed to each of values shown in Table 2 by changing the mesh size of the porous membrane body. The evaluation results are shown in Table 2.

### Comparative Example 2

The respective evaluations were performed in the same manner as in Example 2 except that no porous membrane body was arranged in the internal flow path of the foam discharge container. The evaluation results are shown in Table 2.

**Table 2**

| | Aperture diameter [µm] | Foamability evaluation |
|---|---|---|
| Example 20 | 308 | 5 |
| Example 21 | 215 | 5 |
| Example 22 | 177 | 5 |
| Example 23 | 170 | 5 |
| Example 24 | 165 | 4 |
| Example 25 | 160 | 4 |
| Example 26 | 115 | 3 |
| Example 27 | 77 | 3 |
| Comparative Example 2 | Absent | 1 |

### Examples 28 to 30

The respective evaluations were performed in the same manner as in Example 24 except that the opening ratio of each of the porous membrane bodies arranged in the internal flow path of the foam discharge container was changed to each of values shown in Table 3 by changing the opening ratio of the mesh of the porous membrane body. The evaluation results are shown in Table 3.

**Table 3**

| | Porous membrane body | | Foamability evaluation |
|---|---|---|---|
| | Aperture diameter [µm] | Opening ratio [%] | |
| Example 28 | 160 | 48 | 5 |
| Example 29 | 160 | 43 | 5 |
| Example 30 | 160 | 37 | 4 |

It is found from Tables 1 to 3 that the foaming methods of Examples are superior in foamability to the foaming methods of Comparative Examples.

It is found from the foregoing that the foaming method according to the present invention can be improved in foamability.

A formulation example of the foamable composition according to the present invention is shown below.

### Formulation Example 1

| Raw material name | Blending amount [mass%] |
|---|---|
| Isopropyl myristate | 61.0 |
| Isodecyl neopentanoate | 6.5 |
| 2-Ethylhexyl isononanoate | 7.5 |
| Vegetable oil | 20.0 |
| Trimethylsiloxysilicate | 5.0 |

### Industrial Applicability

According to the present invention, the foaming method and the article each of which is improved in foamability can be provided.

## Claims

1. A foaming method, comprising:
a step (X) of mixing a foamable composition containing a foaming agent (A) having a time required for a reduction in dynamic surface tension thereof to start of 60,000 ms or less, which is measured by the following measurement method 1, and an organic solvent (B), and a gas, and foaming the composition; and
a step (Y) of reducing a size of foam with a porous body:
(Measurement Method 1)
the foaming agent (A) is dissolved in the organic solvent (B) to prepare a solution of the foaming agent (A) having a concentration of 0.5 mass%; then, a dynamic surface tension of the solution of the foaming agent (A) and that of the organic solvent (B) alone at a bubble production time of from 10 ms to 60,000 ms and 25°C are each measured by a maximum bubble pressure method; and then, a first time at which a value obtained by subtracting the dynamic surface tension of the solution of the foaming agent (A) from the dynamic surface tension of the organic solvent (B) alone becomes 0.3 mN/m or more is defined as the time (ms) required for the reduction in dynamic surface tension to start.

2. The foaming method according to claim 1, wherein an amount of a reduction in static surface tension of the foaming agent (A) measured by the following measurement method 2 is 0.5 mN/m or more:
(Measurement Method 2)
the foaming agent (A) is dissolved in the organic solvent (B) to prepare a solution of the foaming agent (A) having a concentration of 0.5 mass%; then, a static surface tension of the solution of the foaming agent (A) and that of the organic solvent (B) alone at 25°C are each measured by a Du Nouy method through lifting of a ring at a speed of 1 mm/min; and then, a value obtained by subtracting the static surface tension of the solution of the foaming agent (A) from the static surface tension of the organic solvent (B) alone is defined as the amount (mN/m) of the reduction in static surface tension.

3. The foaming method according to claim 1 or 2, wherein the foaming agent (A) contains a silicone (A1).

4. The foaming method according to any one of claims 1 to 3, wherein the organic solvent (B) contains at least one kind of water-immiscible solvent (B 1) selected from the group consisting of: a hydrocarbon oil; an ester oil; and a fatty acid.

5. The foaming method according to any one of claims 1 to 4, wherein the shear viscosity of the foamable composition at 25°C and a shear rate of 500 s⁻¹ is 22.0 mPa·s or less.

6. The foaming method according to any one of claims 1 to 5, wherein the porous body has an opening ratio of 30% or more.

7. The foaming method according to any one of claims 1 to 6, wherein the porous body has an aperture diameter of 70 µm or more.

8. The foaming method according to any one of claims 1 to 7, wherein a content of water in the foamable composition is less than 50 mass%.

9. An article, comprising:
a foam discharge container; and
a foamable composition in the foam discharge container,
wherein the foamable composition contains a foaming agent (A) having a time required for a reduction in dynamic surface tension thereof to start of 60,000 ms or less, which is measured by the following measurement method 1, and an organic solvent (B), and
wherein a foam discharge mechanism of the foam discharge container includes a porous body:
(Measurement Method 1)
the foaming agent (A) is dissolved in the organic solvent (B) to prepare a solution of the foaming agent (A) having a concentration of 0.5 mass%; then, a dynamic surface tension of the solution of the foaming agent (A) and that of the organic solvent (B) alone at a bubble production time of from 10 ms to 60,000 ms and 25°C are each measured by a maximum bubble pressure method; and then, a first time at which a value obtained by subtracting the dynamic surface tension of the solution of the foaming agent (A) from the dynamic surface tension of the organic solvent (B) alone becomes 0.3 mN/m or more is defined as the time (ms) required for the reduction in dynamic surface tension to start.

10. The article according to claim 9, wherein an amount of a reduction in static surface tension of the foaming agent (A) measured by the following measurement method 2 is 0.5 mN/m or more:
(Measurement Method 2)
the foaming agent (A) is dissolved in the organic solvent (B) to prepare a solution of the foaming agent (A) having a concentration of 0.5 mass%; then, a static surface tension of the solution of the foaming agent (A) and that of the organic solvent (B) alone at 25°C are each measured by a Du Nouy method through lifting of a ring at a speed of 1 mm/min; and then, a value obtained by subtracting the static surface tension of the solution of the foaming agent (A) from the static surface tension of the organic solvent (B) alone is defined as the amount (mN/m) of the reduction in static surface tension.

11. The article according to claim 9 or 10, wherein the foaming agent (A) contains a silicone (A1).

12. The article according to any one of claims 9 to 11, wherein the organic solvent (B) contains at least one kind of water-immiscible solvent (B1) selected from the group consisting of: a hydrocarbon oil; an ester oil; and a fatty acid.

13. The article according to any one of claims 9 to 12, wherein the shear viscosity of the foamable composition at 25°C and a shear rate of 500 s⁻¹ is 22.0 mPa·s or less.

14. The article according to any one of claims 9 to 13, wherein a content of water in the foamable composition is less than 50 mass%.

15. The article according to any one of claims 9 to 14, wherein the foam discharge container is a non-gas type foam discharge container.

16. The article according to any one of claims 9 to 15, wherein the porous body has an opening ratio of 30% or more.

17. The article according to any one of claims 9 to 16, wherein the foam discharge container is a pump foamer container, a squeeze foamer container, or a trigger spray container.
